# EUROPEAN PATENT APPLICATION

(11) **EP 0 879 882 A1**
(43) Date of publication of application: **25.11.1998**
(21) Application number: 97308557.4
(22) Date of filing: 27.10.1997
(51) Int. Cl.: C12N 15/12, C07K 14/47, A61K 38/17, C12Q 1/68, C07K 16/18

(54) **ATG-1100 (AIF-1-Gamma), a splice variant of AIF-1/RC-9**

(30) Priority: 22.05.1997 US 862167
(71) Applicant: SMITHKLINE BEECHAM CORPORATION, Philadelphia Pennsylvania 19103 (US)
(72) Inventor: Douglas, Stephen A., SmithKline Beecham Pharma., King of Prussia, Pennsylvania 19406 (US)
(74) Representative: Crump, Julian Richard John

(57) **Abstract**

ATG-1100 (AIF-1-gamma) polypeptides and polynucleotides and methods for producing such polypeptides by recombinant techniques are disclosed. Also disclosed are methods for utilizing ATG-1100 (AIF-1-gamma) polypeptides and polynucleotides in the design of protocols for the treatment of atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses, among others, and diagnostic assays for such conditions.

## Description

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to the allograft inflammatory factor-1 family, hereinafter referred to as ATG-1100 (AIF-1-gamma). The invention also relates to inhibiting or activating the action of such polynucleotides and polypeptides.

### BACKGROUND OF THE INVENTION

Advanced coronary, cerebral and peripheral atherosclerosis results in heart attacks and strokes, the primary causes of death in the U.S., Europe and Japan. Nonsurgical treatment of occlusive vascular disease was first described in 1964 and was later adapted for Percutaneous Transluminal Coronary Angioplasty (PTCA). Subsequently, PTCA, and more recently intravascular stent placement, have become prefered alternatives to coronary artery bypass surgery for the treatment of angina and myocardial infarction and can now be used in high risk patients to treat complex lesions with multiple foci in both proximal and distal coronary artery segments. The number of procedures performed annually has grown steadily, exceeding 750,000 per year in the U.S. alone. With an average cost of $15,000 per procedure, PTCA and stent placement account for approximately $10 billion of medical costs in the U.S. Although procedural advances have led to a high primary success rate and low incidence of acute complications, the long term efficacy of PTCA is significantly diminished by restenosis. This process, the direct result of trauma to the vessel wall, results in vascular reocclusion in approximately 40% of patients within ∼6 months post procedure. Almost three quarters of these subjects require repeat angioplasty, stent placement or bypass surgery. Since the cost of these revascularization procedures is estimated to be as high as $4 billion a year, even a modest reduction in restenosis rate by 25% has been estimated to save up to $1 billion annually.

Although cardiologists have two decades worth of experience in performing PTCA, the development of pharmacological adjuncts to coronary angioplasty has met with marginal success e.g. anticoagulant (heparin) and antiplatelet drugs (aspirin, ticlodipine and ReoPro [c7E3]). Thus, restenosis still constitutes a major unmet clinical need in modern interventional cardiology (Douglas, 1996).

The precise molecular mechanisms which underlie the resultant formation of a restenotic lesion are complex and poorly understood. Nevertheless, this dynamic response involves several distinct cellular events. The initial response to injury is primarily inflammatory in nature and, following endothelial cell disruption and platelet activation, involves leukocytes (primarily monocyte-derived macrophages and T-lymphocytes) which release numerous cytokines/chemokines and growth factors in response to injury. These factors, derived from either infiltrating or resident leukocytes are seminal to the local inflammatory response. Secondary to these initial events, the principal cellular component of the lesion, the vascular smooth muscle cell, undergoes an initial phase of mitogenesis following which these cells migrate from the tunica media towards the site of injury. Once in the tunica intima/subendothelial space, these cells undergo profound replication and elaborate large amounts of extracellular matrix. As such, these cells begin to form a neointima which, in time, matures into a plaque large enough to compromise lumen caliber and cause restenosis.

However, as mentioned above, there is no efficacious pharmacological adjunct to prevent clinical restenosis and as such we have attempted to identify molecular targets involved in the pathogenesis of this disease. As such, not only do such gene products represent opportunities for pharmacological intervention (either by conventional small nonpeptide antagonists or by the development of specific neutralizing antibodies) but they also represent potential diagnostic markers of the etiology of such disorders.

Using the technique of differential display reverse transcription polymerase chain reaction (Liang & Pardee, 1992), RC-9 cDNA, a 424bp polynucleotide expressed sequence tag, was identified as being differentially expressed in the rat carotid artery as a consequence of balloon angioplasty (Autieri et al., 1995). Differential upregulation of RC-9 mRNA associated with the fibroproliferative vascular remodeling process characteristic following vascular trauma was confirmed in this model by Northern blot and this partial sequence was used to screen a rat testes cDNA library in order to identify full length rat RC-9 sequence. Once identified, this full length clone was used to search the ATG database and a human homologue was identified (ATG-705; SBC P50420P - U.S. Patent application for "BART-1, a novel balloon angioplasty responsive transcript and uses therefor" [Our Ref: SBC94USA) and SBC P50420-1 - Continuation-in-part patent application for "Use of RC-9 in diagnosis and treatment of proliferative arterial disease" [used for foreign filing, Our Ref: SBC94APCT]). An identical full length rat (GenBank Accession U17919) and human (GenBank Accession U19713) sequence has been identified by Utans et al. (1995, 1996) with potential utility in the treatment and/or diagnosis of allograft rejection/accelerated transplant atherosclerosis (WO 95/17506). More recently, Autieri (1996) has published a polynucleotide seqeuence purported to be human AIF-1 (GenBank Accession U49392). A closely homologous rat (GenBank Accession D82069) sequence has been described in monocytic cells by Imai et al. (1996) and an unpublished human sequence has been deposited in GenBank (Accession D86438).

This indicates that the Allograft inflammatory factor-1 family has an established, proven history as therapeutic targets. Clearly there is a need for identification and characterization of further members of Allograft inflammatory factor-1 family which can play a role in preventing, ameliorating or correcting dysfunctions or diseases, including, but not limited to, atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses.

### SUMMARY OF THE INVENTION

In one aspect, the invention relates to ATG-1100 (AIF-1-gamma) polypeptides and recombinant materials and methods for their production. Another aspect of the invention relates to methods for using such ATG-1100 (AIF-1-gamma) polypeptides and polynucleotides. Such uses include the treatment of atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses, among others. In still another aspect, the invention relates to methods to identify agonists and antagonists using the materials provided by the invention, and treating conditions associated with ATG-1100 (AIF-1-gamma) imbalance with the identified compounds. Yet another aspect of the invention relates to diagnostic assays for detecting diseases associated with inappropriate ATG-1100 (AIF-1-gamma) activity or levels.

### DESCRIPTION OF THE INVENTION

### Definitions

The following definitions are provided to facilitate understanding of certain terms used frequently herein.
"ATG-1100 (AIF-1-gamma) 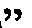 refers, among others, generally to a polypeptide having the amino acid sequence set forth in SEQ ID NO:2 or an allelic variant thereof.
"ATG-1100 (AIF-1-gamma) activity or ATG-1100 (AIF-1-gamma) polypeptide activity 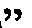 or 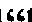 biological activity of the ATG-1100 (AIF-1-gamma) or ATG-1100 (AIF-1-gamma) polypeptide 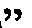 refers to the metabolic or physiologic function of said ATG-1100 (AIF-1-gamma) including similar activities or improved activities or these activities with decreased undesirable side-effects. Also included are antigenic and immunogenic activities of said ATG-1100 (AIF-1-gamma).
"ATG-1100 (AIF-1-gamma) gene 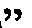 refers to a polynucleotide having the nucleotide sequence set forth in SEQ ID NO:1 or allelic variants thereof and/or their complements.
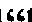 Antibodies 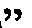 as used herein includes polyclonal and monoclonal antibodies, chimeric, single chain, and humanized antibodies, as well as Fab fragments, including the products of an Fab or other immunoglobulin expression library.
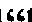 Isolated 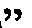 means altered 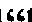 by the hand of man 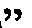 from the natural state. If an 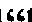 isolated 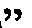 composition or substance occurs in nature, it has been changed or removed from its original environment, or both. For example, a polynucleotide or a polypeptide naturally present in a living animal is not 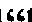 isolated, 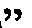 but the same polynucleotide or polypeptide separated from the coexisting materials of its natural state is 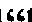 isolated 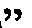 , as the term is employed herein.
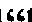 Polynucleotide 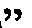 generally refers to any polyribonucleotide or polydeoxribonucleotide, which may be unmodified RNA or DNA or modified RNA or DNA. 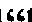 Polynucleotides 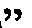 include, without limitation single- and double-stranded DNA, DNA that is a mixture of single- and double-stranded regions, single- and double-stranded RNA, and RNA that is mixture of single-and double-stranded regions, hybrid molecules comprising DNA and RNA that may be single-stranded or, more typically, double-stranded or a mixture of single- and double-stranded regions. In addition, 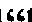 polynucleotide 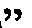 refers to triple-stranded regions comprising RNA or DNA or both RNA and DNA. The term polynucleotide also includes DNAs or RNAs containing one or more modified bases and DNAs or RNAs with backbones modified for stability or for other reasons. 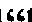 Modified 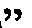 bases include, for example, tritylated bases and unusual bases such as inosine. A variety of modifications has been made to DNA and RNA; thus, 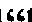 polynucleotide 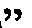 embraces chemically, enzymatically or metabolically modified forms of polynucleotides as typically found in nature, as well as the chemical forms of DNA and RNA characteristic of viruses and cells. 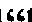 Polynucleotide 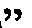 also embraces relatively short polynucleotides, often referred to as oligonucleotides.
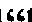 Polypeptide 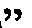 refers to any peptide or protein comprising two or more amino acids joined to each other by peptide bonds or modified peptide bonds, i.e., peptide isosteres. 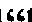 Polypeptide 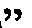 refers to both short chains, commonly referred to as peptides, oligopeptides or oligomers, and to longer chains, generally referred to as proteins. Polypeptides may contain amino acids other than the 20 gene-encoded amino acids. 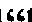 Polypeptides 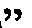 include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al*., 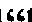 Analysis for protein modifications and nonprotein cofactors 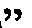 , *Meth Enzymol* (1990) 182:626-646 and Rattan *et al*., 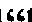 Protein Synthesis: Posttranslational Modifications and Aging 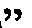 , *Ann NY Acad Sci* (1992) 663:48-62.
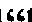 Variant 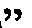 as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.
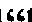 Identity 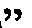 is a measure of the identity of nucleotide sequences or amino acid sequences. In general, the sequences are aligned so that the highest order match is obtained. 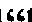 Identity 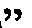 *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term 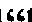 identity 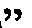 is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al*., *Nucleic Acids Research* (1984) 12(1):387), BLASTP, BLASTN, FASTA (Atschul, S.F. *et al*., *J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to ATG-1100 (AIF-1-gamma) polypeptides. The ATG-1100 (AIF-1-gamma) polypeptides include the polypeptide of SEQ ID NO:2; as well as polypeptides comprising the amino acid sequence of SEQ ID NO: 2; and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within ATG-1100 (AIF-1-gamma) polypeptides are polypeptides having the amino acid sequence which have at least 80% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% are highly preferred. Preferably ATG-1100 (AIF-1-gamma) polypeptide exhibit at least one biological activity of ATG-1100 (AIF-1-gamma).

The ATG-1100 (AIF-1-gamma) polypeptides may be in the form of the 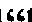 mature 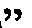 protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the ATG-1100 (AIF-1-gamma) polypeptides are also included in the invention. A fragment is a polypeptide having an amino acid sequence that entirely is the same as part, but not all, of the amino acid sequence of the aforementioned ATG-1100 (AIF-1-gamma) polypeptides. As with ATG-1100 (AIF-1-gamma) polypeptides, fragments may be 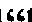 free-standing, 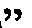 or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of ATG-1100 (AIF-1-gamma) polypeptide. In this context 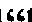 about 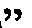 includes the particularly recited ranges larger or smaller by several, 5, 4, 3, 2 or 1 amino acid at either extreme or at both extremes.

Preferred fragments include, for example, truncation polypeptides having the amino acid sequence of ATG-1100 (AIF-1-gamma) polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate ATG-1100 (AIF-1-gamma) activity, including those with a similar activity or an improved activity, or with a decreased undesirable activity. Also included are those that are antigenic or immunogenic in an animal, especially in a human.

Preferably, all of these polypeptide fragments retain the biological activity of the ATG-1100 (AIF-1-gamma), including antigenic activity. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The ATG-1100 (AIF-1-gamma) polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotids of the Invention

Another aspect of the invention relates to ATG-1100 (AIF-1-gamma) polynucleotides. ATG-1100 (AIF-1-gamma) polynucleotides include isolated polynucleotides which encode the ATG-1100 (AIF-1-gamma) polypeptides and fragments, and polynucleotides closely related thereto. More specifically, ATG-1100 (AIF-1-gamma) polynucleotide of the invention include a polynucleotide comprising the nucleotide sequence set forth in SEQ ID NO:1 encoding a ATG-1100 (AIF-1-gamma) polypeptide of SEQ ID NO: 2, and polynucleotide having the particular sequence of SEQ ID NO:1. ATG-1100 (AIF-1-gamma) polynucleotides further include a polynucleotide comprising a nucleotide sequence that has at least 80% identity to a nucleotide sequence encoding the ATG-1100 (AIF-1-gamma) polypeptide of SEQ in NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most: preferred. Also included under ATG-1100 (AIF-1-gamma) polynucleotides are a nucleotide sequence which has sufficient identity to a nucleotide sequence contained in SEQ ID NO:1 to hybridize under conditions useable for amplification or for use as a probe or marker. The invention also provides polynucleotides which are complementary to such ATG-1100 (AIF-1-gamma) polynucleotides.

ATG-1100 (AIF-1-gamma) of the invention is structurally related to other proteins of the allograft inflammatory factor-1 family, as shown by the results of sequencing the cDNA of Table 1 (SEQ ID NO:1) encoding human ATG-1100 (AIF-1-gamma). The cDNA sequence of SEQ ID NO:1 contains an open reading flame (nucleotide numbers 132 to 410) encoding a polypeptide of 93 amino acids of SEQ ID NO:2. The amino acid sequence of Table 2 (SEQ ID NO:2) has about 100% identity (using TBlastN) in 93 amino acid residues with Human allograft inflammatory factor-1 (U. Utans et al., Transplantation 61: 1387-1392, 1996). Furthermore, AIF-1-gamma is 100% identical to human iba-1 over 93 amino acids (Accession number D86438; direct sequence submission to DDBJ, EMBL and GenBank). AIF-1-gamma is 91% identical to both rat iba-1 and rat MRF-1 over 93 amino acids (Y. Imai et al., Biochem. Biophys. Res. Commun., 224: 855-862, 1996; Accession number AB000818, direct submission to DDBJ, EMBL and GenBank, respectively) The nucleotide sequence of Table 1 (SEQ ID NO:1) has about 100% identity (using BlastN) in 282 nucleotide residues with human allograft inflammatory factor-1 (U. Utans et al., Transplantation 61: 1387-1392, 1996). Furthermore, ATG- 1100/AIF-1-gamma is 100% identical with human iba-1 over 282 nucleotides (Accession number D86438; direct sequence submission to DDBJ, EMBL and GenBank) and is 98% identical to the human AIF-1 sequence detailed by M. Autieri (Biochem. Biophys. Res. Commun., 228: 29-37, 1996) over 235 nucleotides.

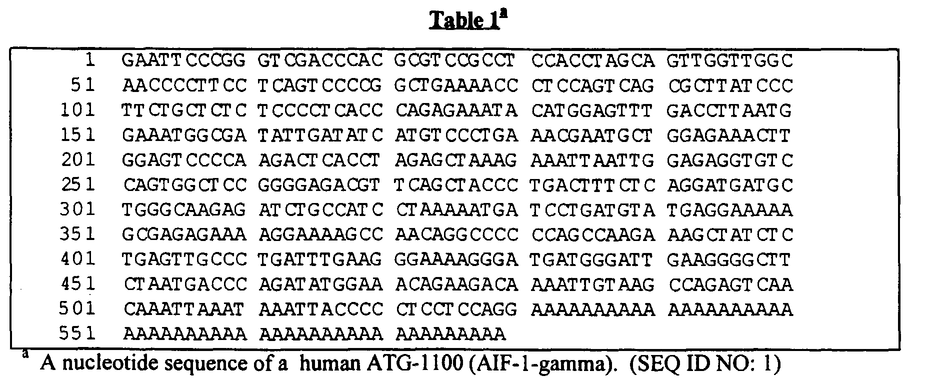

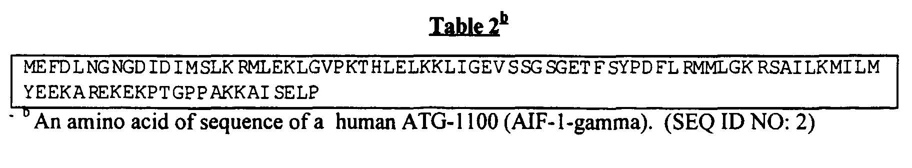

One polynucleotide of the present invention encoding ATG-1100 (AIF-1-gamma) may be obtained using standard cloning and screening, from a cDNA library derived from mRNA in cells of human CD34+ cell/adrenal gland tumor/liver/spleen (chronic lymphocytic leukemia) and other hematopoietic cells using the expressed sequence tag (EST) analysis (Adams, M.D., *et al*. Science (1991) 252:1651-1656; Adams, M.D. *et al*., *Nature,* (1992) *355*:632-634; Adams, M.D., *et al*., *Nature* (1995) 377 Supp:3-174). Polynucleotides of the invention can also be obtained from natural sources such as genomic DNA libraries or can be synthesized using well known and commercially available techniques.

The nucleotide sequence encoding ATG-1100 (AIF-1-gamma) polypeptide of SEQ ID NO:2 may be identical to the polypeptide encoding sequence contained in Table 1 (nucleotide numbers 132 to 410 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of ATG-1100 (AIF-1-gamma) polypeptide, the polynucleotide may include the coding sequence for the mature polypeptide or a fragment thereof, by itself; the coding sequence for the mature polypeptide or fragment in reading flame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al*., *Proc Natl Acad Sci USA* (1989) 86:821-824, or is an HA tag. The polynucleotide may also contain non-coding 5 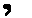 and 3 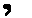 sequences, such as transcribed, non-translated sequences, splicing and polyadenylation signals, ribosome binding sites and sequences that stabilize mRNA.

Further preferred embodiments are polynucleotides encoding ATG-1100 (AIF-1-gamma) variants comprise the amino acid sequence ATG-1100 (AIF-1-gamma) polypeptide of Table 1 (SEQ ID NO:2) in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term 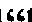 stringent conditions 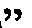 means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof may be used as hybridization probes for cDNA and genomic DNA, to isolate full-length cDNAs and genomic clones encoding ATG-1100 (AIF-1-gamma) polypeptide and to isolate cDNA and genomic clones of other genes that have a high sequence similarity to the ATG-1100 (AIF-1-gamma) gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding ATG-1100 (AIF-1-gamma) polypeptide comprises the steps of screening an appropriate library under stingent hybridization conditions with a labeled probe having the SEQ ID NO: 1 or a fragment thereof; and isolating full-length cDNA and genomic clones containing said polynucleotide sequence. Thus in another aspect, ATG-1100 (AIF-1-gamma) polynucleotides of the present invention further include a nucleotide sequence comprising a nucleotide sequence that hybridize under stringent condition to a nucleotide sequence having SEQ ID NO: 1 or a fragment thereof. Also included with ATG-1100 (AIF-1-gamma) polypeptides are polypeptide comprising amino acid sequence encoded by nucleotide sequence obtained by the above hybridization condition. Such hybridization techniques are well known to those of skill in the art. Stringent hybridization conditions are as defined above or alternatively conditions under overnight incubation at 42^{o}C in a solution comprising: 50% formamide, 5xSSC (150mM NaCl, 15mM trisodium citrate), 50 mM sodium phosphate (pH7.6), 5x Denhardt's solution, 10% dextran sulfate, and 20 microgram/ml denatured, sheared salmon sperm DNA, followed by washing the filters in 0.1x SSC at about 65^{o}C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL*, 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E*. *coli*, *Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al*., *MOLECULAR CLONING A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the ATG-1100 (AIF-1-gamma) polypeptide is to be expressed for use in screening assays, generally, it is preferred that the polypeptide be produced at the surface of the cell. In this event, the cells may be harvested prior to use in the screening assay. If ATG-1100 (AIF-1-gamma) polypeptide is secreted into the medium, the medium can be recovered in order to recover and purify the polypeptide; if produced intracellularly, the cells must first be lysed before the polypeptide is recovered.
ATG-1100 (AIF-1-gamma) polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of ATG-1100 (AIF-1-gamma) polynucleotides for use as diagnostic reagents. Detection of a mutated form of ATG-1100 (AIF-1-gamma) gene associated with a dysfunction will provide a diagnostic tool that can add to or define a diagnosis of a disease or susceptibility to a disease which results from under-expression, over-expression or altered expression of ATG-1100 (AIF-1-gamma). Individuals carrying mutations in the ATG-1100 (AIF-1-gamma) gene may be detected at the DNA level by a variety of techniques.

Nucleic acids for diagnosis may be obtained from a subjects cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled ATG-1100 (AIF-1-gamma) nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al*., *Science* (1985) 230:1242. Sequence changes at specific locations may also be revealed by nuclease protection assays, such as RNase and S1 protection or the chemical cleavage method. See Cotton *et al*., *Proc Natl Acad Sci USA* (1985) 85: 4397-4401. In another embodiment, an array of oligonucleotides probes comprising ATG-1100 (AIF-1-gamma) nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses, through detection of mutation in the ATG-1100 (AIF-1-gamma) gene by the methods described.

In addition, atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses, can be diagnosed by methods comprising determining from a sample derived from a subject an abnormally decreased or increased level of ATG-1100 (AIF-1-gamma) polypeptide or ATG-1100 (AIF-1-gamma) mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an ATG-1100 (AIF-1-gamma) polypeptide, in a sample derived from a host are well-known to those of skill in the art. Such assay methods include radioimmunoassays, competitive-binding assays, Western Blot analysis and ELISA assays.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

AIF-1-gamma appears to be encoded on chromosome 6p21.3 (HLA Class III locus), a region of the human genome known to encode for a wide variety "immune/inflammatory factors" including TNFa/b, HSP-70, B144 and numerous complement proteins (F. Iris et al., Nature Genetics, 3: 137-145, 1993).

The differences in the cDNA or genomic sequence between affected and unaffected individuals can also be determined. If a mutation is observed in some or all of the affected individuals but not in any normal individuals, then the mutation is likely to be the causative agent of the disease.

### Antibodies

The polypeptides of the invention or their fragments or analogs thereof, or cells expressing them can also be used as immunogens to produce antibodies immunospecific for the ATG-1100 (AIF-1-gamma) polypeptides. The term 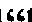 immunospecific 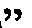 means that the antibodies have substantiall greater affinity for the polypeptides of the invention than their affinity for other related polypeptides in the prior art.

Antibodies generated against the ATG-1100 (AIF-1-gamma) polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al*., *Immunology Today* (1983) 4:72) and the EBV-hybridoma technique (Cole *et al*., MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against ATG-1100 (AIF-1-gamma) polypeptides may also be employed to treat atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses, among others.

### Vaccines

Another aspect of the invention relates to a method for inducing an immunological response in a mammal which comprises inoculating the mammal with ATG-1100 (AIF-1-gamma) polypeptide, or a fragment thereof, adequate to produce antibody and/or T cell immune response to protect said animal from atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses, among others. Yet another aspect of the invention relates to a method of inducing immunological response in a mammal which comprises, delivering ATG-1100 (AIF-1-gamma) polypeptide via a vector directing expression of ATG-1100 (AIF-1-gamma) polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a ATG-1100 (AIF-1-gamma) polypeptide wherein the composition comprises a ATG-1100 (AIF-1-gamma) polypeptide or ATG-1100 (AIF-1-gamma) gene. The vaccine formulation may further comprise a suitable carrier. Since ATG-1100 (AIF-1-gamma) polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The ATG-1100 (AIF-1-gamma) polypeptide of the present invention may be employed in a screening process for compounds which activate (agonists) or inhibit activation of (antagonists, or otherwise called inhibitors) the ATG-1100 (AIF-1-gamma) polypeptide of the present invention. Thus, polypeptides of the invention may also be used to assess identify agonist or antagonists from, for example, cells, cell-free preparations, chemical libraries, and natural product mixtures. These agonists or antagonists may be natural substrates, ligands, receptors, etc., as the case may be, of the polypeptide of the present invention; or may be structural or functional mimetics of the polypeptide of the present invention. See Coligan *et al*., *Current Protocols in Immunology* 1(2):Chapter 5 (1991).

ATG-1100 (AIF-1-gamma) polypeptides are responsible for many biological functions, including many pathologies. Accordingly, it is desirous to find compounds and drugs which stimulate ATG-1100 (AIF-1-gamma) polypeptide on the one hand and which can inhibit the function of ATG-1100 (AIF-1-gamma) polypeptide on the other hand. In general, agonists are employed for therapeutic and prophylactic purposes for such conditions as atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses. Antagonists may be employed for a variety of therapeutic and prophylactic purposes for such conditions as atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses.

In general, such screening procedures may involve using appropriate cells which express the ATG-1100 (AIF-1-gamma) polypeptide or respond to ATG-1100 (AIF-1-gamma) polypeptide of the present invention. Such cells include cells from mammals, yeast, *Drosophila* or *E*. *coli*. Cells which express the ATG-1100 (AIF-1-gamma) polypeptide (or cell membrane containing the expressed polypeptide) or respond to ATG-1100 (AIF-1-gamma) polypeptide are then contacted with a test compound to observe binding, or stimulation or inhibition of a functional response. The ability of the cells which were contacted with the candidate compounds is compared with the same cells which were not contacted for ATG-1100 (AIF-1-gamma) activity.

The assays may simply test binding of a candidate compound wherein adherence to the cells bearing the ATG-1100 (AIF-1-gamma) polypeptide is detected by means of a label directly or indirectly associated with the candidate compound or in an assay involving competition with a labeled competitor. Further, these assays may test whether the candidate compound results in a signal generated by activation of the ATG-1100 (AIF-1-gamma) polypeptide, using detection systems appropriate to the cells bearing the ATG-1100 (AIF-1-gamma) polypeptide. Inhibitors of activation are generally assayed in the presence of a known agonist and the effect on activation by the agonist by the presence of the candidate compound is observed. Standard methods for conducting such screening assays are well understood in the art.

Examples of potential ATG-1100 (AIF-1-gamma) polypeptide antagonists include antibodies or, in some cases, oligonucleotides or proteins which are closely related to the ligands, substrates, receptors, etc., as the case may be, of the ATG-1100 (AIF-1-gamma) polypeptide, e.g., a fragment of the ligands, substrates, receptors, or small molecules which bind to the polypeptide of the present invention but do not elicit a response, so that the activity of the polypeptide is prevented.

### Prophylactic and Therapeutic Methods

This invention provides methods of treating abnormal conditions such as, atherosclerosis, CHF, restenosis, hypertension, fibrotic vasculopathies (diabetes, SLE, AS), cerebrovascular events (e.g. stroke), hematopoietic disorders, ARDS, cancer (particularly leukemias), autoimmune diseases (e.g. HIV-infection and AIDS), fibroproliferative disorders (e.g. psoriasis), inflammatory disease (e.g. RA, Crohn's, IBS), glomerulopathies and disease states, both cardiovascular and non-cardiovascular, which are characterized by aberrant fibroproliferative/inflammatory responses, related to both an excess of and insufficient amounts of ATG-1100 (AIF-1-gamma) polypeptide activity.

If the activity of ATG-1100 (AIF-1-gamma) polypeptide is in excess, several approaches are available. One approach comprises administering to a subject an inhibitor compound (antagonist) as hereinabove described along with a pharmaceutically acceptable carrier in an amount effective to inhibit the function of the ATG-1100 (AIF-1-gamma) polypeptide, such as, for example, by blocking the binding of ligands, substrates, etc., or by inhibiting a second signal, and thereby alleviating the abnormal condition. In another approach, soluble forms of ATG-1100 (AIF-1-gamma) polypeptides still capable of binding the ligand, substrate, etc. in competition with endogenous ATG-1100 (AIF-1-gamma) polypeptide may be administered. Typical embodiments of such competitors comprise fragments of the ATG-1100 (AIF-1-gamma) polypeptide.

In still another approach, expression of the gene encoding endogenous ATG-1100 (AIF-1-gamma) polypeptide can be inhibited using expression blocking techniques. Known such techniques involve the use of antisense sequences, either internally generated or separately administered. See, for example, O 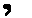 Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al*., *Nucleic Acids Res* (1979) 6:3073; Cooney *et al*., *Science* (1988) 241:456; Dervan *et al*., *Science* (1991) 251:1360. These oligomers can be administered *per se* or the relevant oligomers can be expressed *in vivo*.

For treating abnormal conditions related to an underexpression of ATG-1100 (AIF-1-gamma) and its activity, several approaches are also available. One approach comprises administering to a subject a therapeutically effective amount of a compound which activates ATG-1100 (AIF-1-gamma) polypeptide, i.e., an agonist as described above, in combination with a pharmaceutically acceptable carrier, to thereby alleviate the abnormal condition. Alternatively, gene therapy may be employed to effect the endogenous production of ATG-1100 (AIF-1-gamma) by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo*. For overview of gene therapy, see Chapter 20*, Gene Therapy and other Molecular Genetic*-*based Therapeutic Approaches*, (and references cited therein) in Human Molecular Genetics, T Strachan and A P Read, BIOS Scientific Publishers Ltd (1996). Another approach is to administer therapeutic amount of ATG-1100 (AIF-1-gamma) polypeptides in combination with a suitable pharmaceutical carrier.

### Formulation and Administration

Peptides, such as the soluble form of ATG-1100 (AIF-1-gamma) polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject 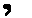 s condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as 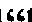 gene therapy 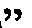 as described above. Thus, for example, cells from a subject may be engineered with a polynucleotide, such as a DNA or RNA, to encode a polypeptide *ex vivo*, and for example, by the use of a retroviral plasmid vector. The cells are then introduced into the subject.

### Examples

The examples below are carried out using standard techniques, which are well known and routine to those of skill in the art, except where otherwise described in detail. The examples illustrate, but do not limit the invention.

### Example 1

When the human RC-9 clone was identified in the ATG database (ATG-705), this sequence (HGS clone ID# 923983) was found to be identical to the human AIF-1 sequence described by Utans et al. (1996). BlastN analysis of public databases also found homology between human RC-9/AIF-1 and a genomic DNA sequence (GenBank Accession Z15025) upstream to that known to encode the human BAT-2 gene i.e. RC-9/AIF-1 was a distinct hitherto uncharacterized gene product lying within a "silent" region of the 90kb HLA class III segment described by Iris et al. (1993).

ATG-705 nucleotide sequence (AIF-1-alpha)(SEQ ID NO: 3):

GGCACGAGAG CCTGCAGACA GAGGCCTCCA GCTTGGTCTG TCTCCCCACC TCTACCAGCA 60
TCTGCTGAGC TATGAGCCAA ACCAGGGATT TACAGGGAGG AAAAGCTTTC GGACTGCTGA 120
AGGCCCAGCA GGAAGAGAGG CTGGATGAGA TCAACAAGCA ATTCCTAGAC GATCCCAAAT 180
ATAGCAGTGA TGAGGATCTG CCCTCCAAAC TGGAAGGCTT CAAAGAGAAA TACATGGAGT 240
TTGACCTTAA TGGAAATGGC GATATTGATA TCATGTCCCT GAAACGAATG CTGGAGAAAC 300
TTGGAGTCCC CAAGACTCAC CTAGAGCTAA AGAAATTAAT TGGAGAGGTG TCCAGTGGCT 360
CCGGGGAGAC GTTCAGCTAC CCTGACTTTC TCAGGATGAT GCTGGGCAAG AGATCTGCCA 420
TCCTAAAAAT GATCCTGATG TATGAGGAAA AAGCGAGAGA AAAGGAAAAG CCAACAGGCC 480
CCCCAGCCAA GAAAGCTATC TCTGAGTTGC CCTGATTTGA AGGGAAAAGG GATGATGGGA 540
TTGAAGGGGC TTCTAATGAC CCAGATATGG AAACAGAAGA CAAAATTGTA AGCCAGAGTC 600
AACAAATTAA ATAAATTACC CCCTCCTCCA AAAAA 635

Detailed sequence alignment of the reverse complementary sequence of human RC-9 and GenBank Accession Z15025 revealed that RC-9/AIF-1 was encoded as 6 distinct exons. In ATG-705, bases 72-74 and 513-515 represent the ATG start and TGA stop codons, respectively, (corresponding to bases to bases 20661-20659 and 19095-19093 of the reverse complement of Z15025). ATG-705 has a 444bp ORE (underlined above) encoding a predicted 147 amino acid residue protein (bases 1-71 represent 5'-UTR and bases 516-629 3'-UTR). In ATG-705, bases 630-635 represent poly(A+) tail. The putative EF-hand/Ca²⁺-binding domain (conserved loop segment, bases 243 to 278) is encoded between exons 4 and 5 and is shown in bold above and below. 
Exon 1 (96 bp)
Reverse complement sequence of ATG-705 base 1 to base 96 aligns with:
GenBank Accession Z15025 base 20732 to base 20637
GGCACGAGAG CCTGCAGACA GAGGCCTCCA GCTTGGTCTG TCTCCCCACC TCTACCAGCA
TCTGCTGAGC TATGAGCCAA ACCAGGGATT TACAGG (SEQ ID NO: 4)

Exon 2 (61 bp)
Reverse complement sequence of ATG-705 base 97 to base 157 aligns with:
GenBank Accession Z15025 base 20471 to base 20411
GAG GAAAAGCTTT CGGACTGCTG AAGGCCCAGC AGGAAGAGAG GCTGGATGAG ATCAACAA
(SEQ ID NO: 5)

Exon 3 (67 bp)
Reverse complement sequence of ATG-705 base 158 to base 224 aligns with:
GenBank Accession Z15025 base 20323 to base 20257
GC AATTCCTAGA CGATCCCAAA TATAGCAGTG ATGAGGATCT GCCCTCCAAA CTGGAAGGCT
TCAAA (SEQ ID NO: 6)

Exon 4 (42 bp)
Reverse complement sequence of ATG-705 base 225 to base 266 aligns with:
 
GenBank Accession Z15025 base 19850 to base 19891
GAGAA ATACATGGAG TTTGACCTTA ATGGAAATGG CGATATT (SEQ ID NO: 7)

Exon 5 (164 bp)
Reverse complement sequence of ATG-705 base 267 to base 430 aligns with:
GenBank Accession Z15025 base 19651 to base 19488
GAT ATCATGTCCC TGAAACGAAT GCTGGAGAAA CTTGGAGTCC CCAAGACTCA
CCTAGAGCTA AAGAAATTAA TTGGAGAGGT GTCCAGTGGC TCCGGGGAGA CGTTCAGCTA
CCCTGACTTT CTCAGGATGA TGCTGGGCAA GAGATCTGCC ATCCTAAAAA T (SEQ ID
NO: 8)

Exon 6 (199 bp)
Reverse complement sequence of ATG-705 base 431 to base 629 aligns with:
GenBank Accession Z15025 base 19178 to base 18980
GATCCTGAT GTATGAGGAA AAAGCGAGAG AAAAGGAAAA GCCAACAGGC CCCCCAGCCA
AGAAAGCTAT CTCTGAGTTG CCCTGATTTG AAGGGAAAAG GGATGATGGG ATTGAAGGGG
CTTCTAATGA CCCAGATATG GAAACAGAAG ACAAAATTGT AAGCCAGAGT CAACAAATTA
AATAAATTAC CCCCTCCTCC (SEQ ID NO: 9)
Intron 1 (exon 1 to 2) spans 165bp GenBank Accession Z15025 base 20636 to base 20472.
Intron 2 (exon 2 to 3) spans 87bp GenBank Accession Z15025 base 20410 to base 20324.
Intron 3 (exon 3 to 4) spans 365bp GenBank Accession Z15025 base 20256 to base 19892.
Intron 4 (exon 4 to 5) spans 178bp GenBank Accession Z15025 base 19849 to base 19652.
Intron 5 (exon 5 to 6) spans 309bp GenBank Accession Z15025 base 19487 to base 19179.

ATG-705 translated protein sequence (putative EF-hand shown in bold):

MSQT RD LQGGKAFGLLKAQQEE RLDEI NKQFLDDPKY SSDEDLP SKLEGFKEKYMEFDLNGNGDIDIM
SLK RMLEKLGVPKTHLELKKLIGEV SSG SGET F SYPD FL RMMLGK RSAI LKMI LMYEEKA REKEKPTG
PPAKKAI SELP   ( SEQ  ID   NO: 10).

Based on the fact that this human cDNA sequence (RC-9/ATG-705/AIF-1) could be aligned in such a manner with a region of genomic sequence, the ATG EST database was searched for the existance of splice variants of human RC-9/AIF-1, lacking one or more of the 6 exons identified above. The parent sequence of human RC-9/AIF-1 (ATG-705), was renamed AIF-1-alpha and the splice variant ATG-1100 was identified.

The first group of ESTs identified appeared to represent, cDNAs derived from mRNAs encoding an N-terminally truncated isoform of RC-9/AIF-1-alpha. These ESTs found in "hematopoietic" (T-lymphocyte and CD34+-) libraries appeared to encode for an mRNA derived from exons 4, 5 and 6. This is exemplified by one full length clone which was isolated and sequenced in its entirety, namely ATG-1100, a sequence given the nomenclature of AIF-1-gamma.

ATG-1100 nucleotide sequence (AIF-1-gamma)

1 GAATTCCCGG GTCGACCCAC GCGTCCGCCT CCACCTAGCA GTTGGTTGGC
51 AACCCCTTCC TCAGTCCCCG GCTGAAAACC CTCCAGTCAG CGCTTATCCC
101 TTCTGCTCTC TCCCCTCACC CAGAGAAATA CATGGAGTTT GACCTTAATG
151 GAAATGGCGA TATTGATATC ATGTCCCTGA AACGAATGCT GGAGAAACTT
201 GGAGTCCCCA AGACTCACCT AGAGCTAAAG AAATTAATTG GAGAGGTGTC
251 CAGTGGCTCC GGGGAGACGT TCAGCTACCC TGACTTTCTC AGGATGATGC
301 TGGGCAAGAG ATCTGCCATC CTAAAAATGA TCCTGATGTA TGAGGAAAAA
351 GCGAGAGAAA AGGAAAAGCC AACAGGCCCC CCAGCCAAGA AAGCTATCTC
401 TGAGTTGCCC TGATTTGAAG GGAAAAGGGA TGATGGGATT GAAGGGGCTT
451 CTAATGACCC AGATATGGAA ACAGAAGACA AAATTGTAAG CCAGAGTCAA
501 CAAATTAAAT AAATTACCCC CTCCTCCAGG AAAAAAAAAA AAAAAAAAAA
551 AAAAAAAAAA AAAAAAAAAA AAAAAAAAA (SEQ ID NO: 1)

(it should also be noted that numerous putative "in frame" ATG start codons are present downstream in the AIF-1-gamma sequence which could potentially act as sites for the intiation of protein translation i.e. this cDNA sequence may act as a template for the translation of numerous truncated forms of the mature AIF-1-gamma protein).

ATG-1100 (AIF-1gamma) translated protein sequence (putative EF-hand shown in bold,
potential downstream "inframe" start codons shown by underlining):
MEFDLNGNGDIDIMSLK RMLEKLGVPKTHLELKKLIGEV SSG SGET F SYPD FL RMMLGK RSAILKMILMYEEKA REKE
KPTGPPAKKAI SELP  (SEQ ID NO: 2).

## Claims

1. An isolated polynucleotide comprising a nucleotide sequence that has at least 80% identity to a nucleotide sequence encoding the ATG-1100 (AIF-1-gamma) polypeptide of SEQ ID NO:2 over its entire length; or a nucleotide sequence complementary to said nucleotide sequence.

2. The polynucleotide of claim 1 which is DNA or RNA.

3. The polynucleotide of claim 1 wherein said nucleotide sequence is at least 80% identical to that contained in SEQ ID NO:1.

4. The polynucleotide of claim 3 wherein said nucleotide sequence comprises the ATG-1100 (AIF-1-gamma) polypeptide encoding sequence contained in SEQ ID NO:1.

5. The polynucleotide of claim 3 which is polynucleotide of SEQ ID NO: 1.

6. A DNA or RNA molecule comprising an expression system, wherein said expression system is capable of producing an ATG-1100 (AIF-1-gamma) polypeptide comprising an amino acid sequence, which has at least 80% identity with the polypeptide of SEQ ID NO:2 when said expression system is present in a compatible host cell.

7. A host cell comprising the expression system of claim 6.

8. A process for producing a ATG-1100 (AIF-1-gamma) polypeptide comprising culturing a host of claim 7 under conditions sufficient for the production of said polypeptide and recovering the polypeptide from the culture.

9. A process for producing a cell which produces a ATG-1100 (AIF-1-gamma) polypeptide thereof comprising transforming or transfecting a host cell with the expression system of claim 6 such that the host cell, under appropriate culture conditions, produces a ATG-1100 (AIF-1-gamma) polypeptide.

10. An ATG-1100 (AIF-1-gamma) polypeptide comprising an amino acid sequence which is at least 80% identical to the amino acid sequence of SEQ ID NO:2 over its entire length.

11. The polypeptide of claim 10 which comprises the amino acid sequence of SEQ ID NO:2.

12. An antibody immunospecific for the ATG-1100 (AIF-1-gamma) polypeptide of claim 10.

13. A method for the treatment of a subject in need of enhanced activity or expression of ATG-1100 (AIF-1-gamma) polypeptide of claim 10 comprising:
(a) administering to the subject a therapeutically effective amount of an agonist to said polypeptide; and/or
(b) providing to the subject an isolated polynucleotide comprising a nucleotide sequence that has at least 80% identity to a nucleotide sequence encoding the ATG-1100 (AIF-1-gamma) polypeptide of SEQ ID NO:2 over its entire length; or a nucleotide sequence complementary to said nucleotide sequence in a form so as to effect production of said polypeptide activity *in vivo*.

14. A method for the treatment of a subject having need to inhibit activity or expression of ATG-1100 (AIF-1-gamma) polypeptide of claim 10 comprising:
(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate, or receptor.

15. A process for diagnosing a disease or a susceptibility to a disease in a subject related to expression or activity of ATG-1100 (AIF-1-gamma) polypeptide of claim 10 in a subject comprising:
(a) determining the presence or absence of a mutation in the nucleotide sequence encoding said ATG-1100 (AIF-1-gamma) polypeptide in the genome of said subject; and/or
(b) analyzing for the presence or amount of the ATG-1100 (AIF-1-gamma) polypeptide expression in a sample derived from said subject.

16. A method for identifying compounds which inhibit (antagonize) or agonize the ATG-1100 (AIF-1-gamma) polypeptide of claim 10 which comprises:
(a) contacting a candidate compound with cells which express the ATG-1100 (AIF-1-gamma) polypeptide (or cell membrane expressing ATG-1100 (AIF-1-gamma) polypeptide) or respond to ATG-1100 (AIF-1-gamma) polypeptide; and
(b) observing the binding, or stimulation or inhibition of a functional response; or comparing the ability of the cells (or cell membrane) which were contacted with the candidate compounds with the same cells which were not contacted for ATG-1100 (AIF-1-gamma) polypeptide activity.

17. An agonist identified by the method of claim 16.

18. An antagonist identified by the method of claim 16.


SEQUENCE LISTING

(1) GENERAL INFORMATION

(i) APPLICANT: SmithKline Beecham Corporation

(ii) TITLE OF THE INVENTION: ATG-1100 (AIF-1-GAMMA), A
NOVEL SPLICE VARIANT OF AIF-1/RC-9

(iii) NUMBER OF SEQUENCES: 10

(iv) CORRESPONDENCE ADDRESS:
(A) ADDRESSEE: SmithKline Beecham, Corporate Intellectual
Property
(B) STREET: Two New Horizons Court
(C) CITY: Brentford
(D) STATE: Middlesex
(E) COUNTRY: GB
(F) POST CODE: TW8 9EP

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER: TO BE ASSIGNED
(B) FILING DATE: 22-MAY-1997
(C) CLASSIFICATION: UNKNOWN

(vii) PRIOR APPLICATION DATA:
(A) APPLICATION NUMBER:
(B) FILING DATE:

(viii) ATTORNEY/AGENT INFORMATION:

(A) NAME: CONNELL, Anthony Christopher
(B) GENERAL AUTHORISATION NUMBER: 5630
(C) REFERENCE/DOCKET NUMBER: GH-70025

(ix) TELECOMMUNICATION INFORMATION:
(A) TELEPHONE: +44 127 964 4395
(B) TELEFAX: +44 18 1975 6294
(C) TELEX:

(2) INFORMATION FOR SEQ ID NO:1:

(i) SEQUENCE CHARACTERISTICS:
(A) LENGTH: 579 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:1:

GAATTCCCGG GTCGACCCAC GCGTCCGCCT CCACCTAGCA GTTGGTTGGC AACCCCTTCC 60
TCAGTCCCCG GCTGAAAACC CTCCAGTCAG CGCTTATCCC TTCTGCTCTC TCCCCTCACC 120
CAGAGAAATA CATGGAGTTT GACCTTAATG GAAATGGCGA TATTGATATC ATGTCCCTGA 180
AACGAATGCT GGAGAAACTT GGAGTCCCCA AGACTCACCT AGAGCTAAAG AAATTAATTG 240
GAGAGGTGTC CAGTGGCTCC GGGGAGACGT TCAGCTACCC TGACTTTCTC AGGATGATGC 300
TGGGCAAGAG ATCTGCCATC CTAAAAATGA TCCTGATGTA TGAGGAAAAA GCGAGAGAAA 360
AGGAAAAGCC AACAGGCCCC CCAGCCAAGA AAGCTATCTC TGAGTTGCCC TGATTTGAAG 420
GGAAAAGGGA TGATGGGATT GAAGGGGCTT CTAATGACCC AGATATGGAA ACAGAAGACA 480
AAATTGTAAG CCAGAGTCAA CAAATTAAAT AAATTACCCC CTCCTCCAGG AAAAAAAAAA 540
AAAAAAAAAA AAAAAAAAAA AAAAAAAAAA AAAAAAAAA 579

(2) INFORMATION FOR SEQ ID NO:2:

(i) SEQUENCE CHARACTERISTICS:
(A) LENGTH: 93 amino acids
(B) TYPE: amino acid
(C) STRANDEDNESS: single
(D) TOPOLOGY: linear
(ii) MOLECULE TYPE: protein

(xi) SEQUENCE DESCRIPTION: SEQ ID NO:2:

Met Glu Phe Asp Leu Asn Gly Asn Gly Asp Ile Asp Ile Met Ser Leu
1               5                   10                  15
Lys Arg Met Leu Glu Lys Leu Gly Val Pro Lys Thr His Leu Glu Leu
            20                  25                  30
Lys Lys Leu Ile Gly Glu Val Ser Ser Gly Ser Gly Glu Thr Phe Ser
        35                  40                  45
Tyr Pro Asp Phe Leu Arg Met Met Leu Gly Lys Arg Ser Ala Ile Leu
    50                  55                  60
Lys Met Ile Leu Met Tyr Glu Glu Lys Ala Arg Glu Lys Glu Lys Pro
65                  70                  75                  80
Thr Gly Pro Pro Ala Lys Lys Ala Ile Ser Glu Leu Pro
                85                  90

(2) INFORMATION FOR SEQ ID NO:3:

(i) SEQUENCE CHARACTERISTICS:
(A) LENGTH: 635 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:3:

GGCACGAGAG CCTGCAGACA GAGGCCTCCA GCTTGGTCTG TCTCCCCACC TCTACCAGCA 60
TCTGCTGAGC TATGAGCCAA ACCAGGGATT TACAGGGAGG AAAAGCTTTC GGACTGCTGA 120
AGGCCCAGCA GGAAGAGAGG CTGGATGAGA TCAACAAGCA ATTCCTAGAC GATCCCAAAT 180
ATAGCAGTGA TGAGGATCTG CCCTCCAAAC TGGAAGGCTT CAAAGAGAAA TACATGGAGT 240
TTGACCTTAA TGGAAATGGC GATATTGATA TCATGTCCCT GAAACGAATG CTGGAGAAAC 300
TTGGAGTCCC CAAGACTCAC CTAGAGCTAA AGAAATTAAT TGGAGAGGTG TCCAGTGGCT 360
CCGGGGAGAC GTTCAGCTAC CCTGACTTTC TCAGGATGAT GCTGGGCAAG AGATCTGCCA 420
TCCTAAAAAT GATCCTGATG TATGAGGAAA AAGCGAGAGA AAAGGAAAAG CCAACAGGCC 480
CCCCAGCCAA GAAAGCTATC TCTGAGTTGC CCTGATTTGA AGGGAAAAGG GATGATGGGA 540
TTGAAGGGGC TTCTAATGAC CCAGATATGG AAACAGAAGA CAAAATTGTA AGCCAGAGTC 600
AACAAATTAA ATAAATTACC CCCTCCTCCA AAAAA 635

(2) INFORMATION FOR SEQ ID NO:4:

(i) SEQUENCE CHARACTERISTICS:
(A) LENGTH: 96 base pairs

(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:4:

GGCACGAGAG CCTGCAGACA GAGGCCTCCA GCTTGGTCTG TCTCCCCACC TCTACCAGCA 60
TCTGCTGAGC TATGAGCCAA ACCAGGGATT TACAGG 96

(2) INFORMATION FOR SEQ ID NO:5:

(i) SEQUENCE CHARACTERISTICS:
(A) LENGTH: 61 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:5:

GAGGAAAAGC TTTCGGACTG CTGAAGGCCC AGCAGGAAGA GAGGCTGGAT GAGATCAACA 60
A 61

(2) INFORMATION FOR SEQ ID NO:6:

(i) SEQUENCE CHARACTERISTICS:
(A) LENGTH: 67 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:6:

GCAATTCCTA GACGATCCCA AATATAGCAG TGATGAGGAT CTGCCCTCCA AACTGGAAGG 60
CTTCAAA 67

(2) INFORMATION FOR SEQ ID NO:7:

(i) SEQUENCE CHARACTERISTICS:
(A) LENGTH: 42 base pairs

(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:7:

GAGAAATACA TGGAGTTTGA CCTTAATGGA AATGGCGATA TT 42

(2) INFORMATION FOR SEQ ID NO:8:

(i) SEQUENCE CHARACTERISTICS:
(A) LENGTH: 164 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:8:

GATATCATGT CCCTGAAACG AATGCTGGAG AAACTTGGAG TCCCCAAGAC TCACCTAGAG 60
CTAAAGAAAT TAATTGGAGA GGTGTCCAGT GGCTCCGGGG AGACGTTCAG CTACCCTGAC 120
TTTCTCAGGA TGATGCTGGG CAAGAGATCT GCCATCCTAA AAAT 164

(2) INFORMATION FOR SEQ ID NO:9:

(i) SEQUENCE CHARACTERISTICS:
(A) LENGTH: 199 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:9:

GATCCTGATG TATGAGGAAA AAGCGAGAGA AAAGGAAAAG CCAACAGGCC CCCCAGCCAA 60
GAAAGCTATC TCTGAGTTGC CCTGATTTGA AGGGAAAAGG GATGATGGGA TTGAAGGGGC 120
TTCTAATGAC CCAGATATGG AAACAGAAGA CAAAATTGTA AGCCAGAGTC AACAAATTAA 180
ATAAATTACC CCCTCCTCC 199

(2) INFORMATION FOR SEQ ID NO:10:

(i) SEQUENCE CHARACTERISTICS:
(A) LENGTH: 147 amino acids
(B) TYPE: amino acid
(C) STRANDEDNESS: single
(D) TOPOLOGY: linear
(ii) MOLECULE TYPE: protein

(xi) SEQUENCE DESCRIPTION: SEQ ID NO:10:

Met Ser Gln Thr Arg Asp Leu Gln Gly Gly Lys Ala Phe Gly Leu Leu
1               5                   10                  15
Lys Ala Gln Gln Glu Glu Arg Leu Asp Glu Ile Asn Lys Gln Phe Leu
            20                  25                  30
Asp Asp Pro Lys Tyr Ser Ser Asp Glu Asp Leu Pro Ser Lys Leu Glu
        35                  40                  45
Gly Phe Lys Glu Lys Tyr Met Glu Phe Asp Leu Asn Gly Asn Gly Asp
    50                  55                  60
Ile Asp Ile Met Ser Leu Lys Arg Met Leu Glu Lys Leu Gly Val Pro
65                  70                  75                  80
Lys Thr His Leu Glu Leu Lys Lys Leu Ile Gly Glu Val Ser Ser Gly
                85                  90                  95
Ser Gly Glu Thr Phe Ser Tyr Pro Asp Phe Leu Arg Met Met Leu Gly
            100                 105                 110
Lys Arg Ser Ala Ile Leu Lys Met Ile Leu Met Tyr Glu Glu Lys Ala
        115                 120                 125
Arg Glu Lys Glu Lys Pro Thr Gly Pro Pro Ala Lys Lys Ala Ile Ser
    130                 135                 140
Glu Leu Pro
145